# EUROPEAN PATENT SPECIFICATION

(11) **EP 2 931 320 B1**
(45) Date of publication and mention of the grant of the patent: **11.10.2017**
(21) Application number: 13826951.9
(22) Date of filing: 12.12.2013
(51) Int. Cl.: A61L 2/26

(54) **CONTAINER FOR STERILIZATION OF MEDICAL ARTICLES**
BEHÄLTER FÜR DIE STERILISATION VON MEDIZINISCHEN ARTIKELN
CONTENANT POUR LA STÉRILISATION D'ARTICLES MÉDICAUX

(30) Priority: 14.12.2012 IT TO20121078
(43) Date of publication of application: 21.10.2015
(73) Proprietor: Eltek S.p.A., 15033 Casale Monferrato (AL) (IT)
(72) Inventor: PETRUZZI, Adriano, I-15033 Casale Monferrato (Alessandria) (IT); GAJ, Renato, I-15033 Casale Monferrato (Alessandria) (IT); PIZZI, Marco, I-15033 Casale Monferrato (Alessandria) (IT)
(74) Representative: Gallarotti, Franco
(86) International application number: PCT/IB2013/060868
(87) International publication number: WO 2014/091448

(56) References cited:
- EP-A1- 2 305 318
- US-A- 4 105 407
- US-A- 4 584 182
- US-A1- 2008 240 979

## Description

### Field of the invention

The present invention relates in general to containers for medical articles and has been developed with particular reference to containers used for sterilization of articles of the aforesaid type, in particular of dental articles, such as heat sterilization and/or sterilization with aeriform fluids and/or in autoclave.

### Prior art

It is known that, after use, various types of medical implements and tools must be subjected to a sterilization cycle, prior to a new use thereof: this is, for example, the case of milling tools used in the dentistry field. It is equally known that, after sterilization, the articles in question are frequently put away for a certain period of time, prior to a subsequent use: however, after removal from the sterilization apparatus, the aforesaid articles run the risk of getting contaminated.

In the above perspective, special containers have been proposed, suitable for containing medical instruments and implements in the course of a sterilization cycle, for example, carried out using autoclave sterilizers, and subsequently keeping them in a protected environment. Containers of this type usually have a main structure that defines a containment space, which can be closed manually in a fluid-tight way, where the article or articles to be sterilized can be laid. Associated to the structure is a movable member, which may be displaced between a position of normal closing and a position of temporary opening, in which the inside of the containment space is isolated from the external environment and in communication therewith, respectively. For the purposes of use, the articles to be sterilized are set in the containment space of the container, and the latter is closed manually and then put inside the treatment chamber of a sterilizer. In the course of the sterilization cycle, the movable member passes from its position of normal closing to the position of temporary opening, thereby enabling the sterilization fluid - for example, steam - to reach the inside of the containment space and carry out its action on the articles. Next, at the end of the sterilization cycle and before the treatment chamber of the sterilizer is re-opened, the movable member again assumes the position of normal closing: in this way, even after removal of the container from the sterilization apparatus, the containment space is sealed with respect to the surrounding environment, thereby preserving the conditions of hygiene of the treated articles.

In containers according to the prior art, the aforesaid movable member is usually represented by an open/close element that belongs to a valve arrangement sensitive to the conditions of pressure that arise in the sterilization chamber in the course of the process, or else belongs to an actuation arrangement operated electrically at predetermined times in the course of the sterilization cycle. Such arrangements are generally complex and costly, at times far from reliable.

EP 2305318 A1, upon which the preamble of claim 1 is based, discloses a sterilization container having a main body, a lid and a safety lock including a thermal switch, designed as a bimetal. The safety lock is arranged to prevent or enable manual opening of the lid depending on the temperature within the container.

US 2008/240979 A1 discloses a system for sterilizing a device, comprising a sterilization chamber, a container having a lid and adapted to receive the device within, a source of sterilizing media connected to the chamber and a mechanism for sealing the lid to the container within the chamber.

US 4105407 A discloses a sterilizing container having a lid, a base and a mechanism for automatically moving the lid into sealing engagement with the base, which mechanism includes pressure responsive means.

US 4584182 A discloses a sterilizing container having a lid which is held open by a support plate carrying a chamber which expands at a predetermined point in a sterilizing cycle to react against the lid, moving the plate outwardly to permit the lid to drop onto the container base. When the container is to be opened, a relief valve relieves the vacuum within the container.

### Object and summary of the invention

In its general terms, the aim of the present invention is to provide a container of the type referred to at the start that is simple and inexpensive to produce, as well as presenting reliable operation. An auxiliary object of the invention is to provide such a container with an improved structure, in particular in order to enable a more efficient fluidic interchange between the inside and the outside of the container. One or more of the above objects are achieved, according to the present invention, by a container for the sterilization of medical articles having the characteristics of Claim 1. Preferred characteristics of the invention are indicated in the sub-claims. The claims form an integral part of the technical teaching provided herein in relation to the invention.

### Brief description of the drawings

Further aims, characteristics and advantages of the invention will emerge clearly from the ensuing detailed description provided with reference to the annexed drawings, which are provided purely by way of explanatory and non-limiting example and in which:
- Figures 1 and 2 are perspective views of a container according to one embodiment of the invention in an open condition and a closed condition, respectively;
- Figures 3 and 4 are perspective views like those of Figures 1 and 2, from a different angle;
- Figure 5 is an exploded view of the container of Figures 1-4;
- Figures 6 and 7 are views at a larger scale of the components of the container of Figure 4;
- Figures 8 and 9 are views like those of Figures 6 and 7, from a different angle;
- Figures 10 and 11 are schematic sections of the container of Figures 1 and 2, respectively;
- Figures 12 and 13 are perspective views, respectively a front view and a rear view, of a container according to a second embodiment of the invention, in an open condition;
- Figures 14 and 15 are schematic representations aimed at exemplifying a modality of use of a container according to the invention;
- Figures 16 and 17 are perspective views of a container according to a third embodiment of the invention, respectively in an open condition and a closed condition;
- Figures 18 and 19 are schematic views in side elevation aimed at exemplifying a mode of use of the container of Figures 16-17; and
- Figures 20 and 21 are perspective views of a container according to a fourth embodiment of the invention, respectively in an open condition and a closed condition.

### Description of preferred embodiments of the invention

Reference to "an embodiment" or "one embodiment" in the framework of the present description is intended to indicate that a particular configuration, structure, or characteristic described in relation to the embodiment is comprised in at least one embodiment. Hence, phrases such as "in an embodiment" or "in one embodiment" and the like that may be present in various points of the present description do not necessarily refer to one and the same embodiment. Furthermore, particular conformations, structures, or characteristics may be combined in any adequate way in one or more embodiments, even different from the ones represented. The references used herein are provided merely for convenience and hence do not define the sphere of protection or the scope of the embodiments. It should moreover be considered that terms such as "bottom" and "top", "up" and "down", "base" and "lid" that may be present in the description refer for simplicity to the arrangements as illustrated in the attached figures, and as such are provided purely by way of non-limiting example.

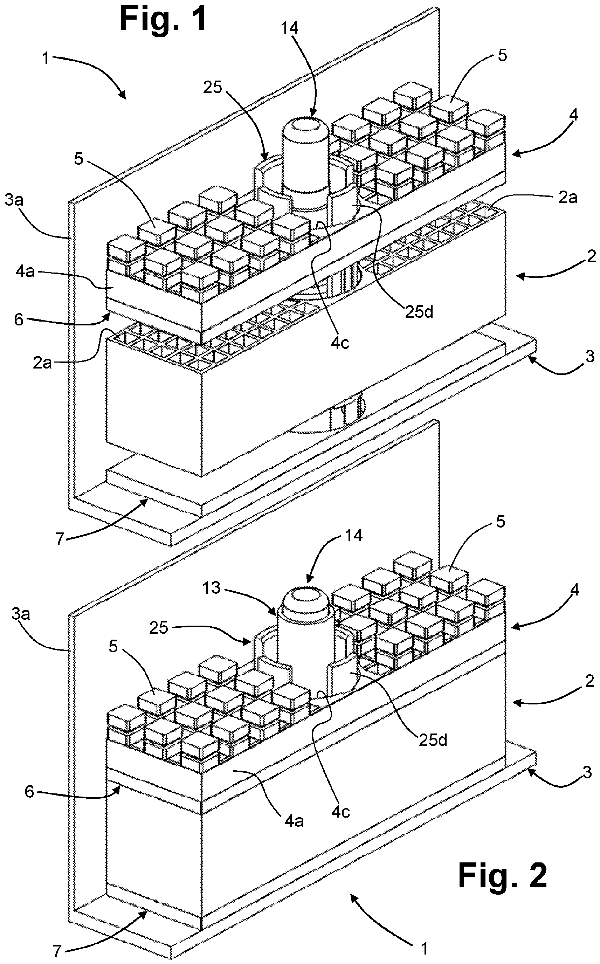

Designated as a whole by 1 in Figures 1-4 is a container according to the invention, such as a container for fluid and/or heat sterilization of medical articles, in particular dental articles, such as milling tools and the like. In a preferred embodiment of the invention, the container 1 is an autoclavable container, i.e., of dimensions such as to enable insertion into an autoclave and capable of supporting the action of an autoclave used for carrying out a cycle of sterilization of the above articles. The container 1 has a main body 2, defining a containment space for the articles that are to undergo sterilization: in the embodiment considered herein, the body 2 has an approximately parallelpipedal shape, with the corresponding containment space that is divided into a plurality of housings or compartments, some of which designated by 2a. The compartments 2a, or more in general the containment space, are open at least in part at the top or bottom longitudinal ends, for the purposes that will emerge more clearly hereinafter. In the case exemplified, at least some of the compartments 2a are open to a different extent at both ends, i.e., sufficiently open at a top end as to enable introduction of articles, and open to a lesser extent at the opposite end, to withhold the aforesaid articles within the compartments, at the same time enabling a circulation or off-flow of a fluid.

The division of the containment space into a plurality of compartments 2a is deemed preferential in embodiments of the type considered herein, provided for sterilization of medical articles having a generally elongated shape, such as precisely milling tools for dentistry: it will be appreciated, however, that the containment space defined by the body 2 could be single, i.e., not divided into a number of compartments or housings. Also the generally prismatic shape of the main body 2 does not constitute an essential characteristic of the invention, said body possibly having also a different shape, for example generally cylindrical, as in one embodiment described hereinafter.

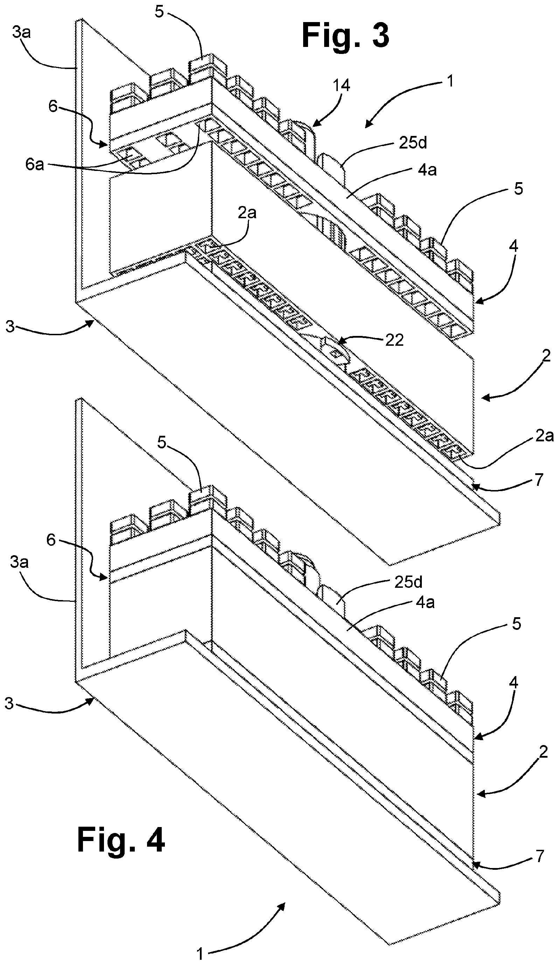

The container 1 comprises at least one wall for opening and closing the containment space defined by the body 2, or the corresponding compartments 2a, at a corresponding longitudinal end (as in the case exemplified) or else at a side end. In the example represented, two said walls are provided, preferably in generally opposite positions of the main body 2, here represented by a base and a lid, for opening and closing the containment space defined by the body 2, respectively at a bottom or top of the containment space itself. In the embodiment illustrated, the structure of the container comprises a base 3, which also forms a part for stationary resting of the container 1, with respect to which the body 2 may be displaced in a constrained way, in particular via a movement of linear sliding. As will emerge more clearly hereinafter, for this purpose, in a preferred embodiment, between the base 3 and the body 2 guide means are provided, configured so as to constrain together the main body and the base 3, to enable the two parts in question to assume at least one position where they are set close up to one another (visible, for example, in Figures 2 and 4) and a position where they are set at a distance apart (visible in Figures 1 and 3).

In a particularly advantageous embodiment, the container 1 includes a lid 4 that can be displaced relative to the body 2, in particular with linear sliding: also in this particularly advantageous embodiment guide means are provided, configured for constraining the lid and the main body together in order to carry out relative displacements between an approached position (Figures 2 and 4) and a spaced-apart position (Figures 1 and 3).

It is to be noted that the presence of two walls, here represented by a base 3 and a lid 4, which may both assume two different positions relative to the main body 2 of the container 1, is to be deemed a preferential characteristic, for the purposes that will emerge more clearly hereinafter. In variant embodiments (not represented), just one wall may be provided, for example, just the base 3, with the lid 4 that is replaced by a series of plugs for the compartments 2a, which can be removed manually, or else with the lid that consists of a lid that can be opened and closed manually, and hence independent of an actuation arrangement of the container 1.

From Figure 3 it may be noted how the bottoms of the compartments 2a are not completely open; i.e., they present openings for passage, aimed at facilitating flow and off-flow of a sterilization fluid, as will emerge more clearly hereinafter. In the example considered, in the substantial proximity of the bottom, the compartments 2a have a generally grill-like, or cros-like or holed structure, in any case configured for enabling support within the compartments themselves of the articles to be sterilized, at the same time enabling passage of the sterilization fluid. The main body 2 may be advantageously made of an injection-mouldable thermoplastic material.

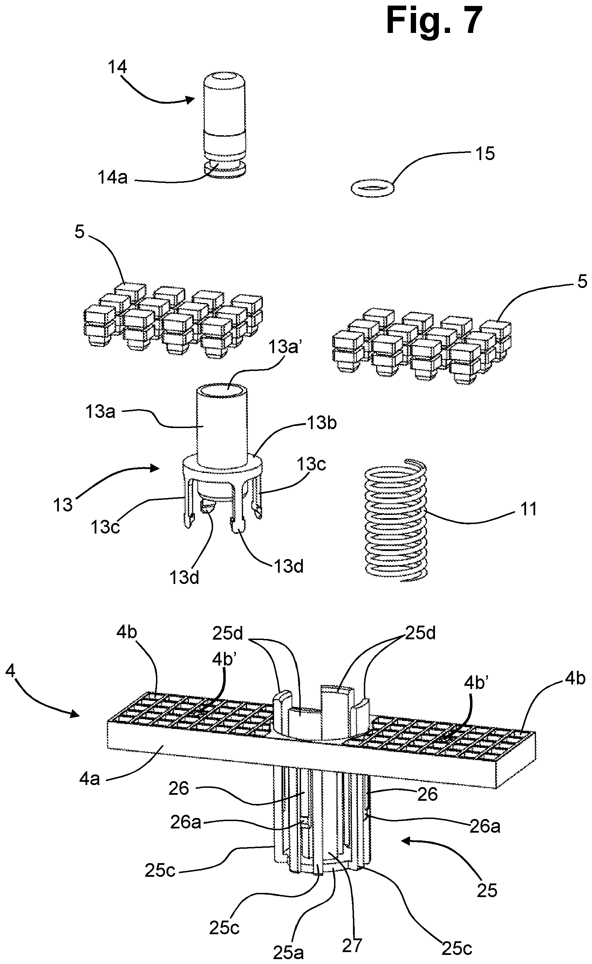

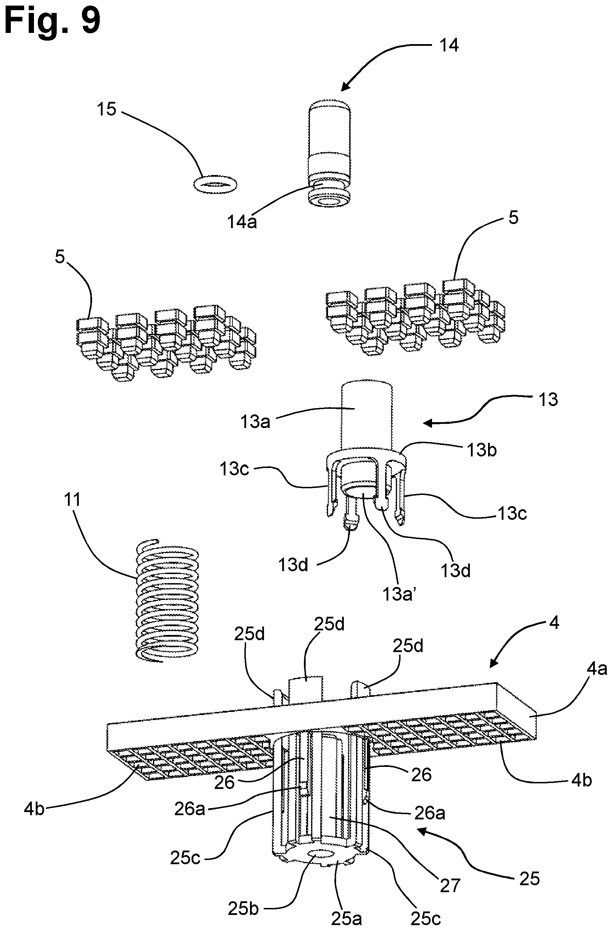

The lid 4 has a structure 4a removably associated to which are closing means 5. In the example represented, as has been said, the main body 2 is divided into a plurality of compartments 2a: for this purpose, the lid 4, which is to close the compartments 2a at the top, has in its structure 4a a plurality of through openings generally aligned to the compartments 2a, some of which designated by 4b in Figures 7 and 9. Positionable on said through openings 4b are the aforesaid closing means, here constituted by plugs 5 that can be removed manually, for example, made of a resilient material and/or designed to provide a seal, such as an elastomer. The structure 4a of the lid 4 has, in the example, a shape generally congruent with the shape of the main body 2a and may be made of an injection-mouldable thermoplastic material; in the example represented, the lid 4 has a generally parallelpipedal shape.

Operative between the main body 2 and the structure 4a of the lid 4 are sealing means, designated by 6, for example, constituted by a gasket made of elastomeric material, here associated to the lid 4. As may be understood, and is clearly visible, for example, in Figure 3, also the gasket 6 has a series of through openings - some of which are designated by 6a - that are axially aligned to the through openings of the structure 4a and to the compartments 2a of the main body 2.

As has been mentioned previously, the structure 4a of the lid 4 is provided with a plurality of through openings 4b, generally aligned to the compartments 2a. It should, however, be pointed out that, with reference to the example illustrated, not all the compartments 2a of the body 2 are to house tools or implements to be sterilized. In the example, the number and position of the compartments 2a that may actually be used are defined by the openings 6a of the gasket 6: as may be clearly noted, for example, from Figures 3, 6 and 8, in fact, the number of the openings 6a of the gasket 6 is decidedly smaller than that of the compartments 2a and of the openings 4b of the structure 4a. The plugs 5 will hence be each time coupled by a user to openings 4b of the structure 4a that are axially aligned to openings 6a of the gasket 6. It may be noted, in this regard, that in the example also the number of plugs 5 is lower than that of the openings 6a of the gasket 6.

Obviously, according to embodiments not represented, it is possible to envisage the same number of plugs 5 and of openings 4b, with the latter that will be axially aligned to respective openings 6a. Obviously, the number of the compartments 2a, of the plugs 5, and of the openings 4b and 6a could even be the same. Of course, it is also possible to provide the structure 4a of the lid 4 so that the openings 4b not to be used are to some extent shielded, for example, by obstructing them partially with a grill-like or similar structure to enable in any case an efficient fluid circulation between the parts of the container 1 (an example of such an embodiment will be exemplified hereinafter).

In general terms, in any case, the presence of a number of compartments 2a greater than those effectively use (i.e., those for which the plugs 5 are provided) is to be deemed preferential in so far as it enables improvement of thermal exchange. More in particular, the compartments 2a that house the implements or tools are adjacent to one or more unoccupied compartments 2a, in which - during the sterilization cycle and the subsequent cooling - the sterilization fluid (for example, steam) can in any case penetrate and then dissipate; in the course of cooling, moreover, in these unoccupied compartments the environmental air can penetrate, thereby favouring cooling.

In a preferred embodiment, also between the base 3 and the lower part of the main body 2 sealing means are operative, designated by 7, for example, a gasket made of elastomeric material, preferably associated to the base 3 in a stationary way.

In general terms, the gasket 6 and/or the gasket 7 have a shape congruent with the face of the body 2 with which it co-operates (here the upper and lower faces of the body 2) and with the corresponding opening/closing wall (here the lid 2 and the base 3). In the example, the gaskets 6 and 7 have a generally parallelpipedal shape, but, as has been said, this shape may be different.

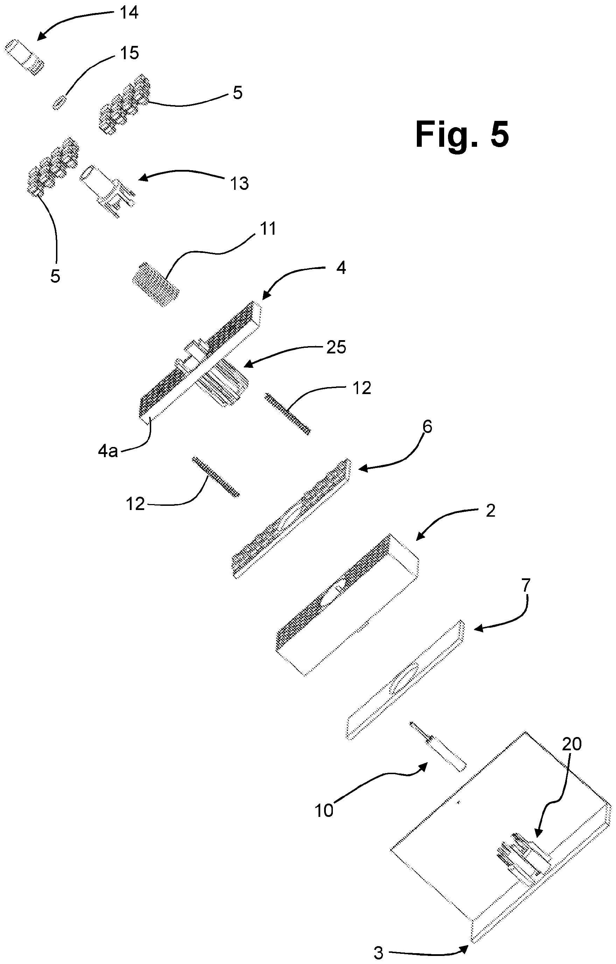

Figure 5 shows in an exploded view the components of the container 1 according to the invention, i.e., the main body 2, the structure 4a of the lid 4, the plugs 5, and the gaskets 6 and 7.

Moreover visible in the figure in question are some components forming part of an actuation and guide arrangement, configured for bringing about passage of the main body 2 and of at least one of the base 3 and the lid 4 between the position where they are set close up to one another and the position where they are set at a distance apart, as referred to previously.

In the example, the actuation arrangement includes an actuator 10 and elastic means 11. In the example considered, since also the lid 4 can be displaced in a way constrained with respect to the main body 2 by means of the aforesaid actuation arrangement, there are preferably - but not necessarily - provided also further elastic means 12. In the figure, moreover designated by 13 is a contrast and/or positioning element for at least the elastic means 11, here represented by a helical spring, the element 13 preferably also having the function of slidably housing a signalling element 14, the functions of which will be clarified hereinafter, to which a ring 15 made of resilient material, of the O-ring type, is associated.

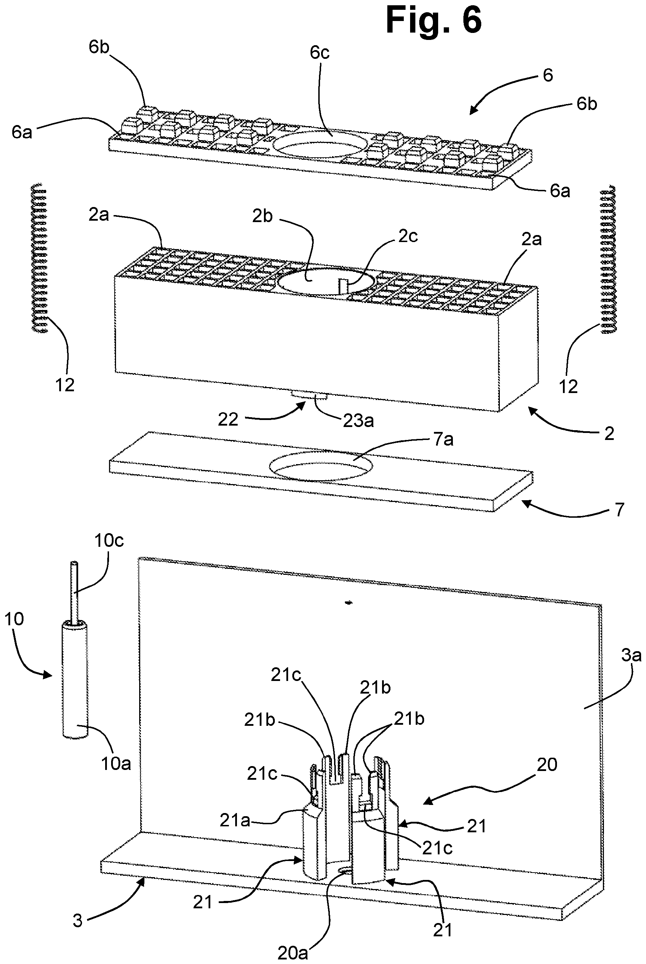

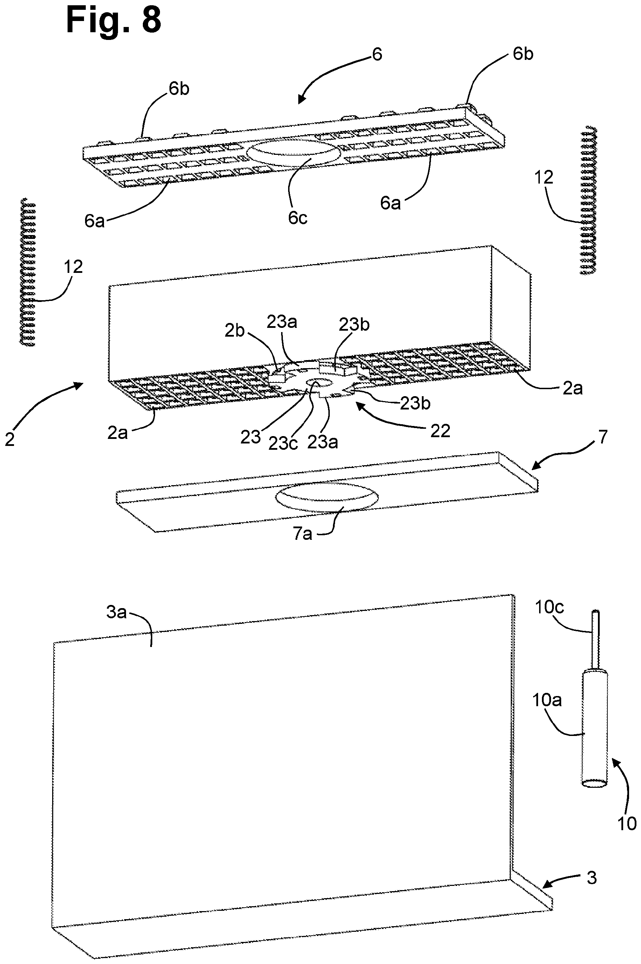

With particular reference to Figures 6 and 8, the structure of the base 3 comprises, in the case exemplified, also an upright wall or resting wall 3a, the presence of which is to be considered optional, albeit preferred for the purposes of positioning of the container 1 according to the invention within a sterilization apparatus such as an autoclave. The base 3 proper has, in a generally central area thereof, a first part of the guide means previously referred to, designated as a whole by 20 and here represented by a formation that comprises a series of upright appendages 21. The upright appendages 21 have, in the case exemplified, a section substantially shaped like the arc of a circle and/or are arranged according to a circumference, at a distance apart from one another. In the example, the appendages 21 each have two portions of different thickness, and in particular a thicker, lower, part and a thinner, upper, part, separated from one another by a transition region 20a, preferably of variable thickness, here defining an inclined plane.

In the non-limiting example, the lower part of the upright appendages 21 has a massive or full structure, and the upper part is configured for defining couplings with the contrast element 13, as will emerge hereinafter: in the example, the upper part of the appendages 21 includes two parallel or specular tabs 21b, which define between them a coupling or engagement seat 21c. Also the base 3 may be advantageously made of an injection-mouldable thermoplastic material. In the example, the formation 20 is defined integrally with the body of the base 3, but there is nothing in principle to rule out configuring it as an additional part fixed to the base itself. The gasket 7 includes a central through hole 7a, preferably having a perimeter at least slightly greater than the perimeter defined by the formation 20 and/or the corresponding appendages 21, which, as has been said, here are arranged according to a circumference.

Preferably defined in the base 3, within the region circumscribed by the upright formations 21, is a seat 20a for positioning the lower end of the actuator 10.

As will emerge more clearly hereinafter, according to the main characteristic of the invention, the actuation arrangement aimed at bringing about passage of the main body 2 and of the at least one wall for closing the containment space (in the example, the wall represented by the base 3 and/or by the lid 4) between the approached position and the spaced-apart position, referred to previously, comprises actuator means sensitive to a temperature of the environment and/or of the sterilization fluid, such as a temperature close or equal to the sterilization temperature.

In the specific case provided purely by way of example, the actuation means comprise a thermo-actuator 10, such as a wax actuator or an actuator whose operation is based upon a material that expands as the temperature increases.

The actuators of the above family are of a conception generally known in the art and do not call for an in-depth description herein. Merely by way of indication, the reader is referred to the document No. EP-A-0940577 for further details as regards the general structure and operation of this family of actuators.

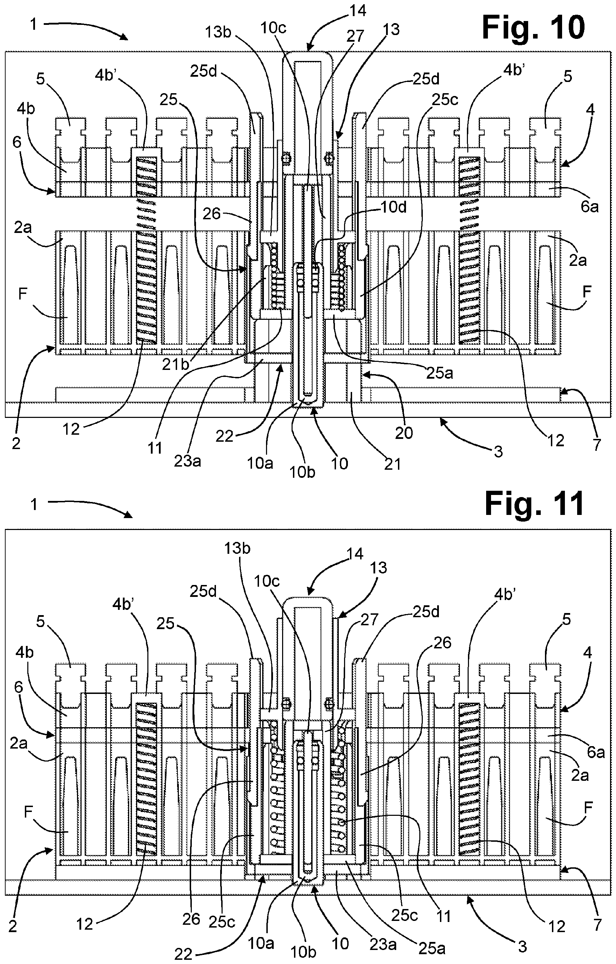

In general terms, the thermo-actuator 10, according to the preferential example illustrated, comprises an actuator body 10a, made of a thermally conductive material, preferably a metal material, for example steel, defining an internal cavity for containment of a material that exapandable as a function of the temperature of the actuator body. Such a cavity is, for example, visible in Figures 10 and 11, where it is designated by 10b. In a preferred embodiment, such as the one represented, the actuator body 10a is made of metal and has relatively thin walls, with a thickness roughly comprised between 0.8 and 1.2 mm in order to enable a rapid response of the device to the temperature of the environment that surrounds it. Very preferably, for this purpose, the shape of the actuator body 10a is at least approximately cylindrical so that the cavity 10b is delimited by substantially uniform walls.

The material that is able to expand as the temperature increases is preferably a wax, but not excluded from the scope of the invention are other materials, such as, for example, particular alcohols or liquids or paraffins having the properties of increasing in volume when subjected to heating. The thermo-actuator 10 then has an axially extended shaft or plunger 10c, having an upper end that is always projecting from the cavity 10b, and preferably from the body 10a, as well as a lower end, opposite to the upper end, which is constantly located within the body 10a or within the corresponding cavity 10b, as may be clearly seen, for example, in Figures 10 and 11. The aforesaid shaft may be in direct contact with the expandable material or separated from the latter via a sealing element or a membrane, for example, made of elastomer. As may be appreciated, the plunger 10c is linearly movable relative to the actuator body 10a as a function of the increase and decrease in volume of the expandable material contained in the cavity 10b (here not indicated).

The structure of the thermo-actuator 10 is then completed by closing means (designated as a whole by 10d only in Figure 10), configured for closing the end of the cavity 10b from which the plunger 10c projects, and for providing a seal with respect thereto, albeit allowing it to slide, so as to prevent any leakage of the thermally expandable material, it preferably comprising at least one rigid element or washer and/or at least one annular sealing element. The thermally expandable material in question is selected from among materials suitable to increase in volume or in any case obtaining a significant increase in volume, such as to move the plunger 10c, at a temperature close to or comprised between the ones typically used in the course of processes of heat sterilization in autoclave or using similar sterilization chambers. By way of indication, the range of temperatures in which expansion of the thermally expandable material occurs is comprised between 50°C and 120°C, preferably comprised between 70°C and 80°C.

As may be appreciated, operation of the thermo-actuator 10 is very simple. When the corresponding actuator body 10a is heated to the temperature comprised in the above range of values, i.e., to a predefined value, the thermally expandable material increases in volume, thereby forcing the plunger 10c towards the outside of the cavity 10b of the actuator body, i.e., varying the overall length or height of the actuator. Actuators of this type are designed to generate significant forces, of the order of kilograms, for example, up to over 10 kg. When the application of heat ceases and when the temperature of the actuator body 10a is gradually brought back to the initial condition, the thermally expandable material progressively reduces its own volume, thereby enabling re-entry of the plunger 10c towards the inside of the cavity, preferably under the action of an external spring. It should be noted that in the figures, except for Figure 11, the thermo-actuator 10 is always represented with the corresponding plunger 10c in an advanced position, but this has merely the purpose of rendering the representation clearer.

The body 2 has, in a generally central area thereof, a cavity 2b, here of a generally cylindrical shape and having a diameter substantially corresponding to the one defined by the appendages 21 of the base 3. Defined along the cylindrical surface of the cavity 2b are axial guides or recesses, one of which is visible in Figure 6, where it is designated by 2c. It should be noted that the upper end of these recesses 2c does not reach the upper edge of the cylindrical cavity 2b, but is at a certain distance (downwards) therefrom. Defined at the lower end of the cavity 2a are guide means 22, which are to co-operate with the guide means 20 of the base 3 for constraining the base itself and the body 2a together to perform a linear movement. These guide means 22 basically comprise a formation that partially closes the cavity 2b in the proximity of its bottom, having a central part 23 with projections 23a set at a distance apart from one another. The shape and position of the radial projections 23a is such that between them depressions or seats 23b are defined, where the upright appendages 21 of the base 3 may be received. The central part 23 then comprises a central through hole 23c, which, in the assembled condition of the container 1, is axially aligned to the seat 22 of the base 3. The formation that constitutes the guide means 22 may be formed integral with the body 2, or else be configured as a distinct component, for example, engaged by snap action in purposely provided seats defined in the body 2, substantially in the proximity of the bottom of its cavity 2a.

Once again with reference to Figures 6 and 8, there may clearly seen the gasket 6 belonging or associated to the lid 4, which cover is here not visible. As has been said, the gasket 6 is preferentially made of an elastic or resilient material, in particular an elastomer, and has a plurality of through openings 6a, in positions substantially corresponding to the through openings of the lid (4b, Figures 7 and 9) referred to previously and to the compartments 2a of the main body 2. In its upper part, the gasket 6 then has coupling parts 6b, in the form of projecting appendages, designed to engage corresponding seats or openings provided in the structure 4a of the lid 4. It may be noted, on the other hand, that the gasket 6 could be overmoulded, with modalities in themselves known, on the structure of the lid 4a, as likewise the gasket 7 may be overmoulded on the base 3. The gasket 6 also has a through opening 6c, in a generally central position, having a perimeter or diameter generally corresponding to or slightly greater than the perimeter or diameter of the cavity 2b of the body 2.

Once again visible in Figures 6 and 8 are the elastic means 12, which are to interact between the main body 2 and the lid 4 in order to urge the latter towards a position where the latter is set at a distance apart from the former. In the example, the elastic means 12 are constituted by helical springs, which may be housed in respective compartments 2a of the body 2, not used for containing articles to be sterilized.

With particular reference to Figures 7 and 9, visible here is the structure 4a of the lid 4, in which the aforementioned openings axially aligned to the compartments 2a of the main body 2 are defined, some of these openings being here designated by 4b. In Figure 7 it may be noted how two of these openings, designated by 4b', are provided with a grill-like or similar formation in order to represent a contrast element for the upper ends of the springs 12 referred to previously (such a formation may also be used, as has been mentioned, for possibly obstructing the openings 4b corresponding to non-usable compartments 2a).

The structure 4a has, in a generally central area thereof, respective guide means, which are to co-operate at least with the guide means of the main body 2, in order to constrain the lid 4 and the body 2 to perform relative displacements between the approached position and the spaced-apart position. In the embodiment exemplified, moreover, the aforesaid guide means are also designed to co-operate with the guide means 20 of the base 3, as will emerge hereinafter. Preferentially, the aforesaid guide means are defined by a shaped element 25, which can be obtained via moulding of thermoplastic material, in particular made of a single piece with the lid 4; said shaped element 25 can, however, be configured as distinct component with respect to the structure 4a, for example, coupled by snap action in a through opening of the structure 4a, by means of hooking elements provided between the structure and the element 25. The element 25 is located on the structure 4a of the lid in a position generally corresponding to the cylindrical cavity 2b of the body 2. The element 25 has a lower base 25a, with a central opening 25b, from which there project in a radial position a plurality of pairs of peripheral upright elements 25c (four pairs, in the example), which extend in a direction substantially orthogonal to the lower base 25a and/or parallel to the axis of the central opening 25b.

The dimensions and position of the upright elements 25c is such that they basically define a circumference or a circular arrangement, substantially corresponding to that of the cylindrical cavity 2b of the body 2, with the pairs of upright elements 25c that are each designed to penetrate into the spaces existing between the appendages 21 of the base 3 (see Figure 6).

The upright elements 25c of each pair are joined at their upper ends, at parts 25d of the element 25 that project at the top from the through opening 4c of the structure 4a. These upper parts 25d of the element 25 preferentially have a section shaped like the arc of a circle and are set at a distance apart from one another to define a circumference substantially equal to the one defined by the cavity 2b and 4c of the body 2 and of the structure 4a, respectively (or the same as the one defined by the outer surfaces of the upright elements 25c).

Once again in Figures 7 and 9, it may be noted how the guide means defined here by the shaped element 25 comprise elastic tabs 26, which project at the bottom from respective parts 26d, in an intermediate position between the two upright elements 25c of a corresponding pair. These tabs 26 present, at their distal ends, respective hooking elements or teeth 26a facing outwards. The shape and dimensions of the elastic tabs 26 with the corresponding teeth 26a is such that they can penetrate into respective grooves 2c (see Figure 6) defined in the cylindrical surface of the cavity 2b of the body 2. As will emerge more clearly hereinafter, the tabs 26 - 26a perform not only a function of linear guide for mutual sliding between the lid 4 and the main body 2, but also an end-of-stroke function, aimed precisely at limiting the maximum stroke allowed for the lid 4 with respect to the body 2. Rising from the base 25a of the element 25 is a compartment, designated by 27 and having a section and dimensions such as to enable housing with relative precision of the body 10a of the thermo-actuator 10, as is clearly visible, for example, in Figures 10 and 11. The seat 27 can have a cylindrical section or, as in the example, may be formed by a plurality of upright walls with cross section shaped like the arc of a circle, which are arranged according to a circumference.

The seat 27 is open at the bottom, at the base 25a, via the opening 25b of Figure 9. Instead, the seat 27 is closed at the top, or in any case has in an upper area a contrast surface for the outer end of the plunger 10c of the thermo-actuator, as is clearly visible once again in Figures 10 and 11. Visible once again in Figures 7 and 9 are the means 11, here represented by a helical spring having a diameter such that it can be inserted into the element 25, in particular in the space existing between the outer surface of the seat 27 and the inner surface of the upright elements 25c and of the tabs 26, as may be seen for example in Figures 10 and 11.

The lower end of the spring 11 is to bear upon the base 25a of the element 25. Instead, the upper end of the spring 11 is to bear upon a corresponding contrast or positioning element, designated as a whole by 13 which is also made, for instance, of injection-moulded thermoplastic material. In the example, the contrast element 13 has a body having a hollow main part 13a, preferably cylindrical. In an intermediate area of the part 13a an outer flange 13b is present, from which there project at the bottom a series of tabs 13c set at a distance apart from one another and having, at the respective lower or distal end, engagement or hooking elements 13d. The shape and position of the tabs 13c and of the corresponding hooking elements 13d, as well as the diameter of the flange 13b, are such that these elements can be inserted between the upper parts 25d of the shaped element 25, with the tabs 13c that can penetrate downwards, until the hooking elements 13d engage in the respective hooking elements or seats 21c defined between the tabs 21b that form the upper part of the appendage 21 of the base 3 (see Figure 6). Consequently, in the assembled condition, the upper end of the spring 111 bears upon the lower surface of the flange 13b of the contrast element 13, in the area circumscribed by the corresponding tabs 13c, the diameter of the flange 13b and of the tabs 13c substantially corresponding to or being slightly smaller than the one defined by the internal surfaces of the top parts 25 of the element 25.

The hollow part 13a of the contrast element 13 defines an axial seat, here of a cylindrical shape, designated by 13 a' only in Figure 7, inside which there can be received from above the signalling element 14, basically constituted by a movable piston or plug at least approximately cylindrical that defines, in its lower area, a seat 14a for a ring made of resilient material 15. The ring 15 basically operates as friction member in order to oppose free sliding of the signalling element 14 within the seat 13a' and can possibly perform also sealing functions.

The seat 13a is open also at the bottom so that a head portion of the seat 27 of the element 25, which houses the thermo-actuator 10 (as is clearly visible, for example, in Figures 10 and 11), can penetrate therein from beneath.

Finally, once again with reference to Figures 7 and 9, the plugs 5 - which, as has been said, are preferably made of an elastomeric material - are visible, these plugs being configured - in particular in their bottom end - for occluding in a fluid-tight way the upper end of the through openings 4b formed in the structure 4a of the lid 4; the upper end of the plugs 5 is instead appropriately shaped to enable manual gripping by an operator for closing and/or opening the openings 4b.

The various components of the container 1 in the embodiment exemplified previously are relatively simple to produce. As already mentioned previously, the base 3 and the main body 2 may be conveniently made of injection-moulded thermoplastic material. A similar technique can be adopted also for the production of the structure 4a of the lid 4 and of the body of the element 25.

Also the bodies of the contrast element 13 and of the signalling element 14 may be conveniently obtained by an operation of moulding of thermoplastic material. Injection-moulding can be adopted also for the production of the gaskets 6 and 7 (even though, as has been said, overmoulding of these elements on the structure 4a of the lid 4 and on the base 3, respectively, is not excluded). Injection-moulding can be adopted also for obtaining the removable plugs 5. The ring 15 is preferably a commercially available component, such as an O-ring, even though it may be of a different shape and type, possibly overmoulded on the signalling element 14, which may possibly be obtained with techniques in themselves known, as likewise the springs 11 and 12.

Assembly of the container 1 is very simple. Associated to the base 3 is the corresponding gasket 7, which is fitted on the formation 20 thanks to the presence of its through opening 7a. Then the main body 2is fitted on the formation 20 of the base 3, by coupling the upright appendages 21 to the depressions 23b (Figure 8) of the formation 22 provided in the lower part of the cylindrical cavity 2b of the body 2. The thermo-actuator 10 is inserted from above so that the bottom of the actuator body 10a positions itself in the seat 22 provided in the base 3.

The shaped element 25, which is fixed with respect to the structure 4 of the lid 4, is inserted from above in the cylindrical cavity 2a of the body 2 so that the body 10a of the actuator 10 with the corresponding plunger 10c can penetrate into the lower opening 25b of the base 25a of the element 25 (and hence into the corresponding seat 27). Obviously, in this assembly step, also the corresponding gasket 6 has already been associated to the structure 4a of the lid 4, and the springs 12 have been inserted in corresponding compartments of the body 2.

The spring 11 can then be inserted from above between the upper parts 25d of the element 25 fixed with respect to the structure 4a of the lid 4 so that the spring itself can penetrate, as has been said, between the space existing between the peripheral uprights 25c and the tabs 26, on one side, and the central seat 27 for the thermo-actuator 10, on the other side, with the lower end of the spring 11 that bears upon the base 25a of the element 25. At this point, between the upper parts 25d of the element 25, the contrast element 13 can be inserted from above so that the hooking elements 13d at the lower ends of the corresponding elastic tabs 13c can couple to the corresponding hooking elements 21c of the upright appendages 21 of the base 3. In this way, bearing upon the lower surface of the flange 13b of the contrast element 13 is the upper end of the spring 11, whilst penetrating into the lower part of the axial seat 13 a' of the contrast element 13 from beneath is an upper-end portion of the seat 27 of the thermo-actuator 10, defined in the element 25. The signalling element 14, equipped with the corresponding resilient O-ring 15, is inserted from above in the seat 13a' itself of the contrast element 13. The plugs 5 are fitted in the corresponding through openings 4b of the structure 4a of the lid 4 in order to occlude them.

It will be appreciated that, in the embodiment exemplified, the guide means represented by the formations 20, 22 and by the element 25 form, together with the contrast element 13, a substantially telescopic coupling between the main body 2, on one side, and the base 3 and/or the lid 4, on the other side. Preferably, the actuator 10 is associated or positioned within the aforesaid telescopic coupling, very preferably but not necessarily substantially coaxial thereto.

As may be understood, in the case exemplified, said telescopic coupling is subject, on one side, to the thrust of the actuator, to bring about a lengthening of the coupling itself, and on the opposite side is subject to the thrust of a spring, compressed during the thrust of the actuator, to bring about shortening of the telescopic coupling when the thrust of the actuator ceases.

The container 1 according to the invention can be advantageously used for preserving the medical articles in a protected environment, which in the example of Figures 10 and 11 are represented by milling tools for dentistry, designated as a whole by F. In this condition of use of the container 1, as "magazine" for the milling tools F, the container itself is generally at ambient temperature, i.e., in a condition of temperature that is not such as to cause a significant increase in volume of the thermo-expandable material inside the thermo-actuator 10. In this condition, as may be understood, for example from Figure 11, the spring 11 constantly forces the lid 4 towards the base 3, thereby also forcing the body 2 towards the base 3, i.e., on the corresponding gasket 7. This occurs on account of the fact that the contrast element 13 is constrained to the base 3, and precisely to the hooking elements 21c defined in the upright appendages 21 of the formation 20, with the spring 11 that is in turn set between the aforesaid contrast element 13 and the element 25 fixed with respect to the lid 4 (in particular, as has been seen, the spring 11 is located between the flange 13b of the contrast element 13 and the base 25a of the element 25 fixed with respect to the structure 4a of the lid 4). Basically, as may be understood from Figure 11, the elastic reaction of the spring 11 pushes the structure 4a of the lid 4, and hence the corresponding gasket 6, against the main body 2, thereby also pushing the main body 2 on the gasket 7 associated to the base 3. In this condition, the springs 12 are kept in a condition of elastic load, the springs themselves tending to facilitate separation of the lid 4 from the main body 2, even though they have a force weaker than that of the spring 11.

In a condition of the device resembling the one represented in Figure 11, the compartments 2a of the body 2 (in any case, the compartments 2a effectively used for housing the implements or milling tools F), are axially aligned to the respective through openings 6a of the gasket 6 and to the through openings 4b of the structure 4a of the body 4, with the plugs 5 that thus close the compartments themselves at the top (or, rather, the corresponding prolongations constituted by the through openings 6a, 4b of the gasket 6 and of the structure 4a).

Thanks to the plugs 5 made of resilient material and to the sealing action exerted by the gasket 6, the compartments 2a are thus closed in a fluid-tight way at the top. The fact that the lower part of the main body 2 is forced by the spring 12 against the lower gasket 7 ensures closing in a fluid-tight way of the compartments 2a also in their lower part. Consequently, in this condition, the compartments 2a are firmly sealed with respect to the external environment, thereby preserving the sterile condition of the milling tools F. It goes without saying that, when it is necessary to use one of the implements or milling tools F, the user merely has to remove one of the plugs 5 and slide out the corresponding milling tools F from the corresponding compartment 2a, for example, by turning the body of the container 1 upside down.

According to a possible variant of the device, it is possible to envisage two series of plugs 5 having different colouring, to be used in combination with a container 1, each colouring indicating the condition of the tool contained in a compartment 2a (for example, red plugs and blue plugs, to be used alternatively to indicate compartments that contain tools F that require or else do not require sterilization). According to this possible variant, after use of a milling tool F, and in view of subsequent sterilization thereof, the milling tool itself can be put back into the compartment 2a, re-closed with a red plug, to distinguish it from compartments with a blue plug, which contain the sterile milling tools. The use of coloured plugs could, however, serve also to identify better the contents of the various sterilized compartments, for example by associating a further colour to each type of milling tool contained therein, in the case of a number of different milling tools or implements housed in a single container according to the invention.

Regardless of the variant just mentioned, in normal use at least two containers according to the invention are preferably provided. The sterilized tools taken out of the first container are used and then laid in the second container in order to facilitate sterilization of even only some milling tools or implements. When all the tools have been removed from the first container, the latter can also be used for receiving other tools that are to undergo sterilization.

The at least one container that is to contain sterilized tools and the at least one container that is to contain tools to be sterilized may be distinguished from one another by suitable indication means, such as the aforesaid signalling element 14. In a possible use, and given that the element 14 may be configured for being fitted in the corresponding seat 13a' or else manually removed therefrom, a user can, after execution of a sterilization step carried out with the first container, fit the element 14 into the aforesaid seat or else remove it precisely to indicate that the tools contained therein have been sterilized; the other container, in which the tools to be sterilized are to be laid, will instead have the element 14 partially removed from the seat 13a' or else present therein, respectively, precisely in order to indicate that it is to be used for carrying out the sterilization cycle. In this perspective, then, it is also possible to provide a kit consisting of two containers 1 and a single signalling element 14, to be displaced, as required, between the two containers of the kit.

In general terms, in any case, there is preferably provided a generic element 14, the function of which is at least that of enabling verification that the components 3-4 have actually been separated in the sterilization step, within the tolerances established for proper sterilization (for example, indication with coloured band), and which optionally may possibly be used for the organizational logic described in the previous paragraphs.

The structure and dimensions of the device according to the invention are in general relatively contained and may enable a convenient use and sterilization of a plurality of containers of this sort, also in order to be able to use a container for each type of tool or implement that is to undergo treatment.

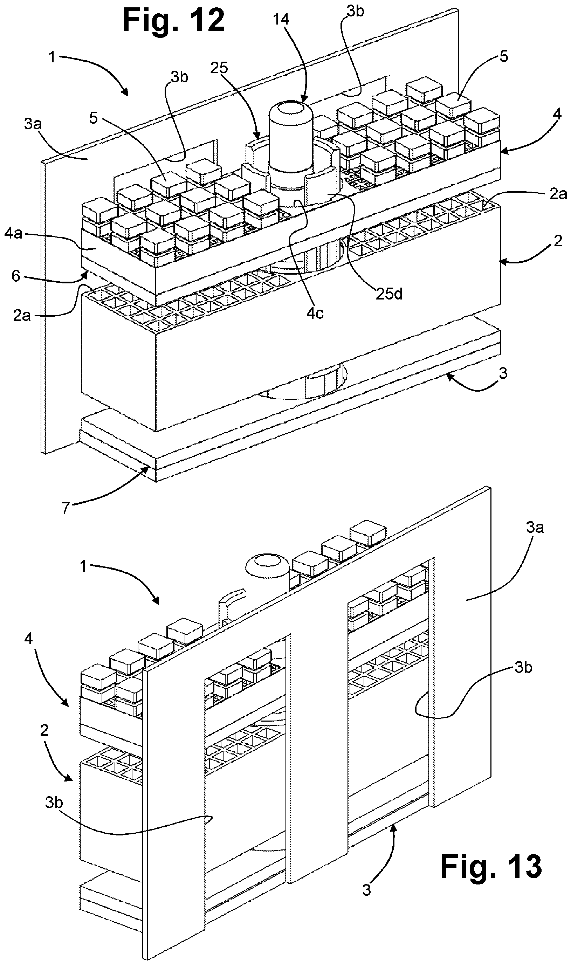

Figures 12 and 13 illustrate an embodiment of the container 1 in which the wall 3a associated to the base 3, and which here is to function as resting surface for the container 1, is provided with one or more openings or windows 3b aimed at facilitating circulation of a fluid present in the sterilization chamber in which the container itself is set. Figures 12 and 13 moreover show how, in a possible embodiment, the base 3a can have a width smaller than that of the aforesaid wall 3a. As already mentioned previously, in possible embodiments, the openings 4b of the structure 4a that are not located at openings 6a of the gasket 6 (or more in general the openings 4b not to be used) may be shielded at least partially. Such a case is exemplified in Figures 12 and 13, where it may be noted how the openings of the structure 4a not occupied by plugs 5 are provided with a grill-like formation, or in any case a shielding designed not to enable insertion of tools but designed to enable circulation of a fluid.

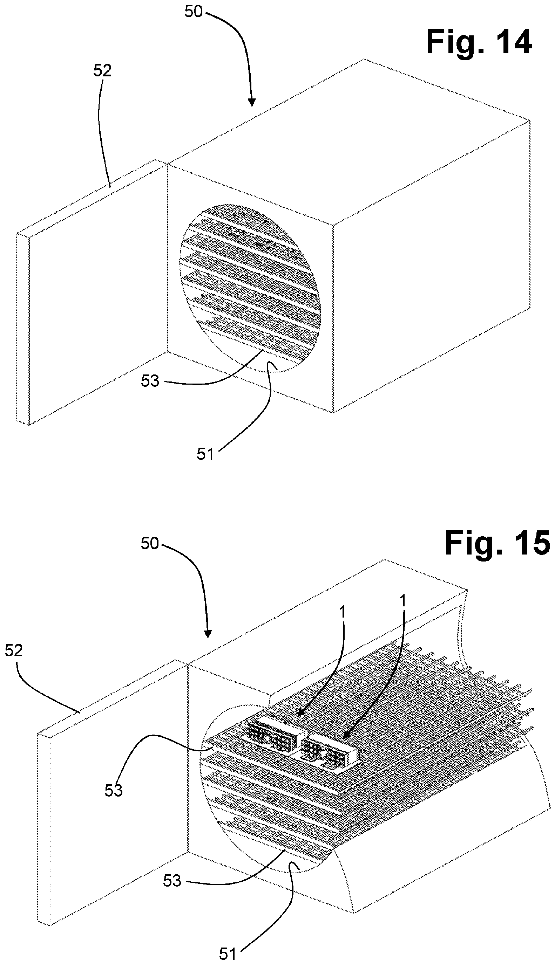

When sterilization of one or more tools F becomes necessary, the latter are set inside corresponding compartments 2a of a container 1. The container 1, or containers 1, are then set within a sterilization chamber, necessary for carrying out a cycle of sterilization of the tools themselves. Preferentially, as already mentioned, the container 1 is an autoclavable container, i.e., one designed to withstand the conditions of temperature/pressure that arise within an autoclave sterilization apparatus. Figures 14 and 15 show, purely by way of example, a possible use in this sense, where designated as a whole by 50 is an autoclave sterilization apparatus, having a sterilization chamber 51, which can be closed in a fluid-tight way by a corresponding hatch 52, and within which shelves 53 are provided - here having a generally grill-like structure - on which to set or hook the containers 1, as may be seen for example in Figure 15 (it should be noted that in this figure the body of the apparatus 50 is partially sectioned and with some shelves 53 removed in order to show the inside of the corresponding sterilization chamber 51). In the example, the two containers 1 housed in the sterilization chamber 51 are of the type illustrated in Figures 12 and 13 and use the wall 3a as part for resting on the corresponding shelf 53 (for requirements of explanation, one of the containers 1 is shown in the open condition and the other in the closed condition). In an application of this sort, the base 3 with the associated wall 3a constitute a stationary part of the container 1, with the body 2 and the lid 4 mobile relative thereto.

As exemplified, the particular shape and/or the contained dimensions of the container 1, preferably essentially parallelpipedal in shape with overall dimensions of approximately 10 x 7 x 3 cm, enables convenient sterilization of a number of devices 1 set alongside one another, arranged on various shelves of an autoclave or other sterilization apparatus.

Following upon start of the sterilization cycle, the temperature within the chamber 51 increases considerably, for example, up to 134°C. This increase in temperature has the effect of bringing about heating of the actuator body 10a, with the consequent increase in temperature of the thermo-expandable material. This material then increases in volume, thereby pushing the plunger 10c towards the outside of the corresponding cavity 10b. As may well be noted in Figures 10 and 11, located in front of the upper end of the plunger 10c is the upper contrast wall (not indicated) of the seat 27 (see Figures 7 and 9), where the thermo-actuator 10 is located. In this way, the plunger 10c progressively pushes the element 25 upwards, and therewith the structure 4a of the lid 4 with the elements annexed thereto (the gasket 6 and the plugs 5) and with the contrast element 13, which instead remains stationary in so far as it is constrained to the base 3. As will be appreciated in particular from Figure 11, the action of the plunger 10c - with the consequent thrust upwards of the element 25 and of the lid 4 associated thereto - brings about compression of the spring 11. At a certain point of the travel of the plunger 10c, raising of the lid 4 also brings about raising of the main body 2 with respect to the base 3 and to the corresponding gasket 7, on account of the fact that the tabs 26 of the element 25, with the corresponding engagement teeth 26a, are engaged in the grooves 2c defined within the cylindrical cavity 2b of the main body 2 (see Figure 6). In practice, when the teeth 26a reach the upper end of the aforesaid grooves 2c, the structure 4a, or rather the tabs 26 fixed with respect thereto, tend to pull the body 2 upwards with respect to the base 3. In this step, the springs 12 perform the function of facilitating uniform opening between the lid 4 and the main body 2 and/or keeping them in a condition set at a distance apart from one another. It will be appreciated that, in this step, the tabs 26-26 coupled to the corresponding grooves 2c form, together with the contrast element 13, end-of-stroke means designed to limit the maximum stoke allowed for the relative movement between the lid 4 and the body 2 and hence also between the container 2 and the base 3.

As may be understood from a comparison between Figures 11 and 10, in the course of the linear movement of the various parts, the seat 27 that houses the thermo-actuator 10 penetrates progressively within the axial seat defined in the contrast element 13 (the seat 13a' of Figure 7): this brings about progressive raising of the signalling element 14, which thus projects further from the aforesaid seat with respect to the original position, visible in Figure 11.

In the condition exemplified in Figure 10, then, the compartments 2a of the main body 2 are open at both ends and are hence in fluid communication with the environment inside the sterilization chamber 51 of the apparatus 50. This favours circulation or in any case gaseous interchange with the sterilization fluid, for example, represented by steam, which can hence penetrate and flow freely in the compartments 2a in order to carry out its own sterilization function. At the end of the sterilization cycle, the temperature within the sterilization chamber 51 starts to drop progressively. This entails progressive reduction of the temperature of the actuator body 10a, and thus a corresponding reduction of temperature of the thermo-sensitive material. This material then starts to contract, i.e., to reduce in volume, thereby enabling progressive re-entry of the plunger 10c towards the inside of the cavity 10b of the actuator body 10a. This action of progressive re-entry is evidently favoured by the action of the spring 11, which tends to push the lid 4 and the main body 2 downwards. The time of return of the thermo-sensitive material to the initial conditions of volume is not immediate, but occurs with a certain delay, which substantially depends upon the time that the actuator body 10a takes to reach the temperature of solidification of the thermo-sensitive material 10b, for example, of the order of minutes from when heating of the sterilization environment has ceased. Consider, in this regard, that the above delay also depends upon the thermal inertia of the sterilization chamber, i.e., upon the thermal inertia with which the heat accumulated in the chamber is dissipated. In general terms, in an actuator of the type considered herein, the time of re-entry of the plunger determined by just the thermal inertia of the actuator body and of the expandable material is variable between approximately 1 and 3 minutes at ambient temperature: in the case of use of such an actuator within a sterilization chamber that cools off relatively slowly, this re-entry time is consequently greater, except in the case where the cooling chamber is provided with some sort of cooling system. The above-mentioned delay also enables draining of possible residue of fluid on the outside of the compartments 2a, through the passages formed in the bottom thereof.

In a preferred embodiment, the sequence of the translations of the components of the device during exit of the plunger 10c is the following (for example, assuming a useful stroke of the plunger itself of 16 mm): for the first half of the stroke of exit (the first 8 mm) there is required a compression of the spring 11 and a corresponding recession of the lid 4 with respect to the base 3 and the body 2, with the latter that remains in contact with the base 3 (i.e., with the gasket 7) as a result of the springs 12; in the second half of the stroke of exit of the plunger 10c (the next 8 mm) there occurs a further compression of the spring 11, with increase of the recession of the lid 4 with respect to the base 3 (16 mm in all) and with recession of the body 2 with respect to the base 3 (i.e., by 8 mm) as a result of the engagement teeth 26a of the element 25 associated to the body 4 and the corresponding contrast elements defined by the grooves 2c of the body 2. The body 2 is thus at the same distance (namely, 8 mm) both from the lid 4 and from the base, and in this situation remains there throughout the sterilization time, facilitating as much as possible flow of the sterilization fluid in the housings 2a. In the subsequent step of re-entry of the plunger 10c the spring 11 extends: in the first half of the stroke of re-entry (the first 8 mm) the body 2 comes to rest again on the base 3 (i.e., on its gasket 7) as a result of the springs 11 and 12, whilst in the second half of the stroke of re-entry (the next 8 mm) the lid 4 (i.e., its gasket 6) comes to rest again on the body 2, as a result of the spring 11, which has a force greater than the sum of the forces of the springs 12, which in this step act in a direction opposite to that of the spring 11.

Advantageously, the thermal inertia of the actuator 10 also enables compensation of a momentary absence of electric power supply of the sterilization apparatus. The sterilization chamber of such an apparatus is generally able to maintain the temperature reached for a relatively long time, even in the absence of electric power supply: in this way, even in certain anomalous conditions or in the absence of electric power supply, the actuator 10 in any case enables the container 1 to be kept open, i.e., enables continuation of sterilization of the tools.

After the time of cooling deemed adequate, or in any case at the end of the sterilization step and/or return of the autoclave to ambient temperature, then, the container 1 has returned to the initial condition of Figure 11, where the compartments 2a are closed and sealed, with the milling tools F now sterilized inside. The container 1 can hence be removed from the sterilization chamber 51 after prior opening of the hatch 52. Of course, the elastic reaction of the spring 11 is much stronger than that of the springs 12 so as to guarantee re-closing of the various parts 2-4 of the container 1.

The presence of the resilient O-ring 15 associated to the signalling element 14 counters free re-entry of the element itself into the seat 13 a' of the contrast element 13. Consequently, in other words, after a sterilization cycle, the signalling element 14 partially exits from the seat 13a' and remains in any case in the raised condition, as may be seen for example in Figure 10. In this way, the body of the signalling element 14, which preferably at least in part has a different colouring (for example green) from other surrounding elements of the container (such as the lid 4 and/or the plugs 5 and/or the element 25), constitutes an element visually indicating effective opening of the various parts 2-4 of the container 1 during a step heating to a predefined temperature; i.e., it signals that the sterilization step has been carried out properly. Obviously, in view of a subsequent use of the container 1 for carrying out a sterilization step, the user merely has to push the signalling element 14 manually downwards until it is brought into the condition shown in Figure 11.

As has been said, the indication provided by the element 14 may be designed to identify better the containers 1 with sterilized tools or implements, in particular with the signalling element 14 at least in part extracted and/or with a differentiated colouring visible, and/or to identify better the containers with tools or implements still to be sterilized, in particular with the signalling element 14 at least in part retracted and/or with the differentiated colouring not visible.

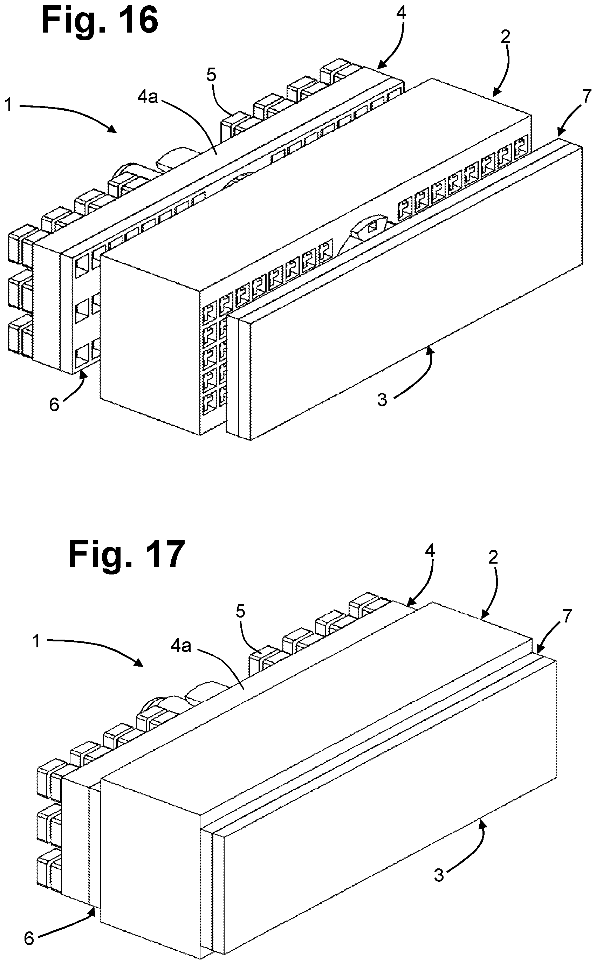

Illustrated in Figures 16 and 17 is a further embodiment of a container 1 according to the invention, the structure of which is basically distinguished from that of the containers of the previous figures by the absence of the wall 3a and by the fact that the main body 2 constitutes or comprises a rest, preferably having at least one dimension of lateral encumbrance larger than that of the at least one opening/closing wall of the containment space (in the case exemplified, the walls represented by the base 3 and the lid 4).

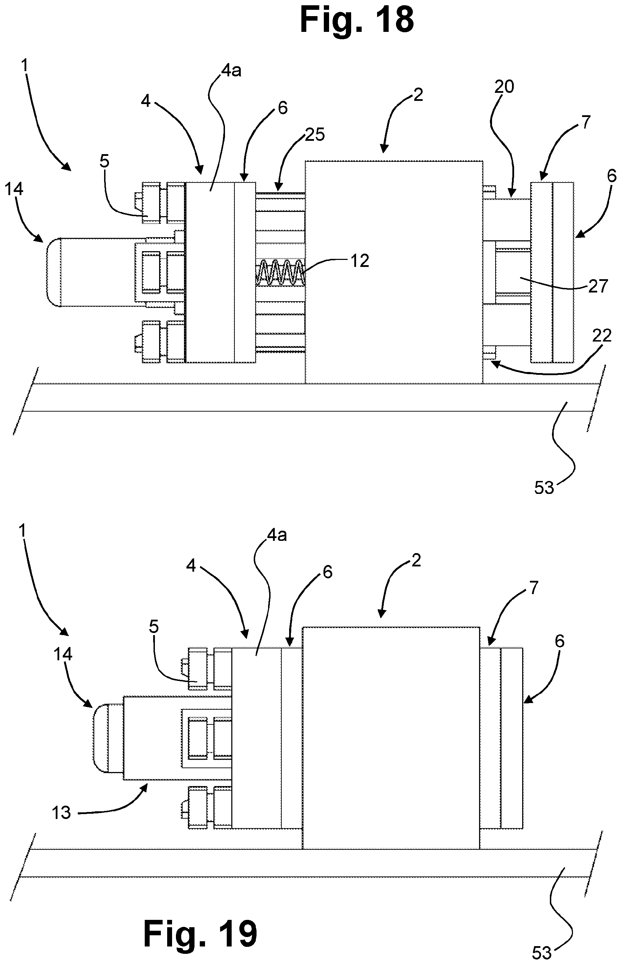

Preferentially, for such a case, the body 2 has a generally polyhedral shape, said shape being, in the case exemplified, substantially parallelpipedal. As may be seen in the subsequent Figures 18 and 19, in this embodiment the container 1 can be set resting on a surface, such as a shelf 53, for supporting a sterilization apparatus by means of or in a position corresponding to one the two lateral faces of the body 2 that define the aforesaid rest and/or the aforesaid dimension of lateral encumbrance, here the major lateral faces of the body 2.

The body 2 and/or at least one of the corresponding faces may possibly be associated to, or provided with, a resting flange, also in order to increase the resting surface. In this embodiment, then, the body 2 defining the containment space constitutes a stationary part of the container 1, with respect to which the at least one opening/closing wall, such as the base 3 and/or the lid 4, can be displaced. Considering such an embodiment or else the example of application referred to in Figures 14 and 15, it will thus be appreciated that terms such as "base" or "lid", "bottom" and "top", "up" and "down" have a purely relative nature and are provided by way of non-limiting example. Obviously, when not used in a sterilization cycle, the container 1 of Figures 16-19 can be rested on a generic surface exploiting the base 3 (a similar consideration applies also to the case of the containers 1 exemplified in Figures 1- 15).

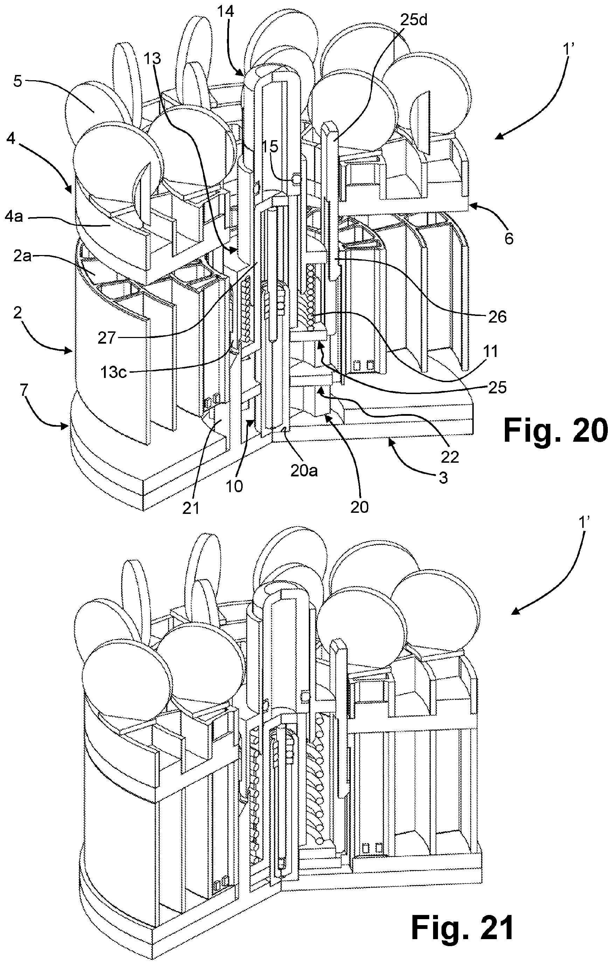

Represented by partially sectioned perspective views in Figures 20 and 21 is a further possible embodiment of a container according to the invention. The container of this second embodiment, designated as a whole by 1', is not described in detail here, in so far as it in effect uses elements having functions similar to those of the elements already described with reference to the previous figures, of which they retain the same reference numbers. In fact, the only significant differences of the container 1' with respect to the containers 1 described previously are represented by the absence - in the second embodiment - of the springs 12 and by a different shape of some components, and specifically of the base 3, of the main body 2, and of the lid 4, with the corresponding gaskets 6 and 7, which here have a generally circular or cylindrical shape. Also the shape of the plugs 5 is in this case different, however, merely as regards the fact that they have a gripping flap. The cross section of the compartments 2a here substantially has the shape of the sector of a circle, or a wedge shape, or a trapezial shape, preferably with profiles that are at least in part curved. For the rest, the construction and operation of the various elements are similar to the ones described previously.

From the foregoing description there emerge clearly the characteristics of the present invention, as likewise its advantages. The container 1, 1' described is simple and economically advantageous to produce, as well as easy and fast to assemble. Its operation is extremely reliable, being based upon the use of precise guide means and of an actuation member that is able to develop a significant power in order to guarantee the function of opening/closing between two or more parts of the container itself. Opening and closing of the container are moreover performed automatically, without the need for sources of electric power supply and hence free from drawbacks linked to possible interruptions of electrical supply.

The actuation arrangement aimed at enabling approach/recession of the at least one opening/closing wall is extremely simple and inexpensive to produce, and enables opening and closing of ample sections of passage to be obtained, which in the limit may correspond to an entire face of the body defining the containment space, thereby improving circulation of the sterilization fluid in the container, in a more uniform and rapid way.

The fact that, in a preferred, but non-exclusive, embodiment of the invention, relative movements are obtained between the main body 2 and the base 3, as well as between the main body 2 and the lid 4, enables opening of the containment space for the articles to be sterilized at both ends, thereby improving further the effectiveness of the sterilization cycle.

The container according to the invention enables an efficient fluidic exchange and/or heat exchange between the outside and the inside and vice versa, in the course of the sterilization cycle and of a subsequent cooling cycle. As has been mentioned, this is possibly improved, in one embodiment of the invention, thanks to the fact that the compartments that are to receive the articles to be sterilized are adjacent to one or more unoccupied compartments, in which the sterilization fluid first and the cooling fluid after can flow.

The fluidic exchange and/or heat exchange occurs in particular from the outside towards the inside in the sterilization step, whereas it occurs from the inside towards the outside during cooling, to speed up dissipation of the fluid and/or of heat.

The fact that, in a preferred embodiment, the container includes a signalling element that can be actuated by the same actuation that brings about opening of the container makes it possible to provide clear indications on effective opening of the containment space in the course of the sterilization step, and hence of effectiveness of the treatment, and - in addition or as an alternative - an indication aimed at facilitating distinction between containers that contain implements or tools that have undergone sterilization and containers that contain implements or tools that are to undergo sterilization (in particular, also in view of the possibility of manual switching of the aforesaid signalling element).

It is clear that the container described by way of example may undergo numerous variations and modifications by persons skilled in the branch, without thereby departing from the scope of the invention as defined in the annexed claims.

The presence of a lid operated by the actuation arrangement is to be understood preferable, but not indispensable. As has been mentioned, and with simple modifications that appear clearly to the person skilled in the branch, the lid 4 could even be omitted, for example, by providing a lid that can be removed manually or a series of plugs like the ones previously designated by 5, for occluding directly the upper end of respective housings or compartments defined in the main body 2.

With reference to the example of Figures 1-11, the base 3 proper can have a width smaller than the one represented, i.e., substantially corresponding to that of the body 2 and/or of the corresponding gasket 7, as simplified only in Figure 13: this in order to improve circulation of the sterilization flow.

The movement of opening and closing, described preferentially with reference to a mechanism of a telescopic and/or linear type could also be of a different type, such as a movement of opening/closing at least in a rotating and/or angled part, for example, by envisaging constraints or fulcra between the container 2 and the base 3 and/or the lid 4.

In embodiments not represented, the actuator means sensitive to the temperature of the sterilization environment may comprise, in addition or as an alternative to a thermo-actuator of the type exemplified, a different thermo-actuator, such as an actuation member of the bimetallic type or made of a shape-memory alloy.

## Claims

1. Medical article sterilization container, particularly for dental articles, comprising
- a main body (2), defining a containment space (2a) for articles to be sterilized (F),
- at least one wall (3, 7; 4, 6) for opening and closing the containment space (2a) at at least one end of the containment space (2a),
- guide means (2b, 2c, 20, 22, 25), configured for constraining the main body (2) and the at least one wall (3, 7; 4, 6) to perform relative displacements between an approached position and a spaced-apart position,
- an actuation arrangement (10-13, 26), which comprises actuator means (10) sensitive to a temperature of the sterilization environment,
**characterized in that** the actuation arrangement (10-13, 26) is configured for causing relative displacements of the main body (2) and the at least one wall (3, 7; 4, 6) between the approached position and the space-apart position.

2. The container according to claim 1, comprising a first said wall (3, 7) and a second said wall (4, 6), and wherein
- the guide means (2b, 2c, 20, 22, 25) comprise
- guide means (20, 22) configured for constraining the main body (2) and the first wall (3, 7) to perform relative displacements between the respective approached and spaced-apart positions, and
- guide means (2b, 2c, 20, 22, 25) configured for constraining the second wall (4, 6) and the main body (2) to perform relative displacements between the respective approached and spaced-apart positions,
- the actuation arrangement (10-13, 26) is configured for causing relative displacement between the main body (2) and the first wall (3, 7) in the respective approached and spaced-apart positions and relative displacement between the second wall (4, 6) and the main body (2) in the respective approached and spaced-apart positions.

3. The container according to claim 1 or claim 2, wherein the actuation arrangement (10-13, 26) comprises coupling or end-of-stroke means (13, 26a), configured for limiting the maximum stroke allowed to the relative movement between the main body (2) and the at least one wall (3, 7; 4, 6).

4. The container according to one or more of the preceding claims, wherein the actuation arrangement (10-13, 26) comprises elastic means (11, 12) designed to urge the main body (2) and the at least one wall (3, 7; 4, 6) in a corresponding approached position or spaced-apart position, such as at least one of
- elastic means (11) designed to urge the main body (2) and at least one said wall (3, 7) in a corresponding approached position, and
- elastic means (12) designed to urge the main body (2) and at least one said wall (4, 6) in a corresponding spaced-apart position.

5. The container according to any one of the preceding claims, wherein the actuator means comprise a thermal actuator (10) including
- an actuator body made of thermally conductive material (10a), into which a cavity (10b) is defined to contain a material expandable according to the temperature of the actuator body (10a), preferably a wax or a paraffin,
- a shaft or plunger (10c), having one end, protruding from an end of the cavity (10b), and a second end, within the cavity (10b), the shaft or plunger (10c) being linearly movable relative to the actuator body (10a) according to the increase and the reduction of volume of the expandable material,
- closing means (10d), for closing said end of the cavity (10b) of the actuator body (10a) and obtain a seal with respect to the shaft or plunger (10c).

6. The container according to one of the preceding claims, wherein the guide means (2b, 2c, 20, 22, 25) comprise guide means (20) fixed with respect to the first wall (3, 7) and engaged in a slidable way with guide means (22) fixed with respect to the main body (2).

7. The container according to one of the preceding claims, wherein the guide means (2b, 2c, 20, 22, 25) comprise guide means (25) fixed relative to the second wall (4, 6) and engaged in a slidable way with at least one of guide means (20) fixed with respect to the first wall (3, 7) and guide means (2b, 2c, 22) fixed with respect to the main body (2).

8. The container according to one of the preceding claims, comprising at least one substantially telescopic coupling (20, 22, 25) between the main body (2) and the at least one wall (3, 7; 4, 6).

9. The container according to claim 8, wherein the actuator means (1-13, 26) comprises an actuator (10) housed in or associated to the substantially telescopic coupling (20, 22, 25), preferably in a position substantially coaxial thereto.

10. The container according to any one of the preceding claims, wherein the containment space (2a) has a bottom with passage openings for a sterilizing fluid, the bottom being preferably closable selectively via the at least one wall (3, 7; 4, 6).

11. The container according to any one of the preceding claims, wherein the at least one wall (3, 7; 4, 6) has a structure (4a, 6) to which there are removably associated closing means (5), preferably manual closing means, such as plugs or the like.

12. The container according to any one of the preceding claims, wherein the containment space is divided into a plurality of compartments (2a), where preferably the plurality of compartments (2a) comprises one or more operative compartments, i.e., designed for housing articles (F) to be sterilized, and one or more inoperative compartments, i.e., not designed for housing articles (F) to be sterilized, one or more inoperative compartments being in a position adjacent to at least one operative compartment, very preferably the main body (2) and/or the at least one wall (3, 7; 4, 6) being configured in such a way that the one or more inoperative compartments remains in fluid communication with the outer environment also when the main body (2) and the at least one wall (3, 7; 4, 6) are in a corresponding approached position.

13. The container according to claims 11 and 12, wherein the structure (4a, 6) of the at least one wall (4, 6) has a plurality of through-openings (4b, 6a) axially aligned to respective compartments (2a) of said plurality, there being preferably associated to the through-openings (4a, 6a) respective manually removable plugs (5).

14. The container according to one of the preceding claims, comprising a positioning member (13) coupled to the first wall (3, 7) in a substantially stationary position, the positioning member (13) and the first wall (3, 7) having means for reciprocal coupling (13c, 13d, 21b, 21c), at least one of the main body (2) and the second wall (4, 6) being operatively set between the positioning member (13) and the first wall (3, 7), between the positioning member (13) and at least one of the main body (2) and the second wall (4, 6) there being preferably provided elastic means (11) for urging the main body (2) in a respective position approached to the first wall (3, 7).

15. The container according to one of the preceding claims, also comprising a signalling member (14), preferably designed to signal, particularly in an automatic manner, at least one of an actuation that has occurred of the actuation arrangement, a relative displacement that has occurred between the main body (2) and the at least one wall (3, 7, 4, 6), a sterilization that has occurred, a condition of the container (1), a feature of articles (F) contained in the containment space, the signalling member (14) being preferably displaceable from a first position to a second position following upon passage of the main body (2) and the at least one wall (3, 7, 4, 6) from the approached position to the spaced-apart position, the signalling member (14) being very preferably manually operable to be brought back into the aforesaid first position.

## Patentansprüche

1. Sterilisationsbehälter für medizinische Produkte, insbesondere für zahnärztliche Produkte, mit
- einem Hauptkörper (2), der einen Aufnahmeraum (2a) für zu sterilisierende Produkte (F) begrenzt,
- mindestens einer Wand (3, 7; 4, 6) zum Öffnen und Schließen des Aufnahmeraums (2a) an mindestens einem Ende des Aufnahmeraums (2a),
- einer Führungseinrichtung (2b, 2c, 20, 22, 25), die ausgebildet ist, den Hauptkörper (2) und die mindestens eine Wand (3, 7; 4, 6) zum Ausführen relativer Verschiebungen zwischen einer angenäherten Position und einer beabstandeten Position zu führen,
- einer Betätigungsanordnung (10-13, 26), die eine Aktuatoreinrichtung (10) aufweist, die für eine Temperatur der Sterilisierungsumgebung empfindlich ist,
**dadurch gekennzeichnet, dass** die Betätigungsanordnung (10-13, 26) ausgebildet ist, relative Verschiebungen des Hauptkörpers (2) und der mindestens einen Wand (3, 7; 4, 6) zwischen der angenäherten Position und der beabstandeten Position zu veranlassen.

2. Behälter nach Anspruch 1, der eine erste besagte Wand (3, 7) und eine zweite besagte Wand (4, 6) aufweist, und wobei
- die Führungseinrichtung (2b, 2c, 20, 22, 25) aufweist
- eine Führungseinrichtung (20, 22), die ausgebildet ist, den Hauptkörper (2) und die erste Wand (3, 7) zur Ausführung relativer Verschiebungen zwischen der jeweiligen angenäherten Position und der beabstandeten Position zu führen, und
- eine Führungseinrichtung (2b, 2c, 20, 22, 25), die ausgebildet ist, die zweite Wand (4, 6) und den Hauptkörper (2) zur Ausführung relativer Verschiebungen zwischen der entsprechenden angenäherten Position und der beabstandeten Position zu führen,
- die Betätigungsanordnung (10-13, 26) ausgebildet ist, eine relative Verschiebung zwischen dem Hauptkörper (2) und der ersten Wand (3, 7) in der entsprechenden angenäherten Position und der beabstandeten Position und eine relative Verschiebung zwischen der zweiten Wand (4, 6) und dem Hauptkörper (2) in der entsprechenden angenäherten Position und der beabstandeten Position zu veranlassen.

3. Behälter nach Anspruch 1 oder Anspruch 2, wobei die Betätigungsanordnung (10-13, 26) eine Kopplungs- oder Bewegungsbegrenzungseinrichtung (12, 26a) aufweist, die ausgebildet ist, den maximalen Bewegungsbereich, der für die relative Verschiebung zwischen dem Hauptkörper (2) und der mindestens einen Wand (3, 7; 4, 6) zulässig ist, zu begrenzen.

4. Behälter nach einem oder mehreren der vorhergehenden Ansprüche, wobei die Betätigungsanordnung (10-13, 26) eine elastische Einrichtung (11, 12) aufweist, die so ausgebildet ist, dass sie den Hauptkörper (2) und die mindestens eine Wand (3, 7; 4, 6) in eine entsprechende angenäherte Position oder eine beabstandete Position vorspannt, insbesondere
- eine elastische Einrichtung (11), die zum Vorspannen des Hauptkörpers (2) und der mindestens einen Wand (3, 7) in eine entsprechende angenäherte Position ausgebildet ist, und/oder
- eine elastische Einrichtung (12), die zum Vorspannen des Hauptkörpers (2) und der mindestens einen Wand (4, 6) in eine entsprechende beabstandete Position ausgebildet ist.

5. Behälter nach einem der vorhergehenden Ansprüche, wobei die Aktuatoreinrichtung einen thermischen Aktuator (10) aufweist, mit
- einem Aktuatorkörper, der aus einem wärmeleitenden Material (10a) hergestellt ist, in welchem eine Aussparung (10b) gebildet ist, um ein Material, das sich entsprechend der Temperatur des Aktuatorkörpers (10a) ausdehnen kann, vorzugsweise ein Wachs oder Paraffin, aufzunehmen,
- einem Schaft oder Stößel (10c), der ein Ende hat, das von einem Ende der Aussparung (10b) hervorsteht, und ein zweites Ende in der Aussparung (10b) hat, wobei der Schaft oder Stößel (10c) geradlinig relativ zu dem Aktuatorkörper (10a) gemäß der Ausdehnung oder der Schrumpfung des Volumens des ausdehnbaren Materials bewegbar ist,
- einer Schließeinrichtung (10d) zum Schließen des Endes der Aussparung (10b) des Aktuatorkörpers (10a) und zum Erreichen einer Dichtigkeit in Bezug auf den Schaft oder Stößel (10c).

6. Behälter nach einem der vorhergehenden Ansprüche, wobei die Führungseinrichtung (2b, 2c, 20, 22, 25) eine Führungseinrichtung (20) umfasst, die in Bezug auf die erste Wand (3, 7) fixiert ist und gleitend mit einer Führungseinrichtung (22) im Eingriff ist, die in Bezug auf den Hauptkörper (2) fixiert ist.

7. Behälter nach einem der vorhergehenden Ansprüche, wobei die Führungseinrichtung (2b, 2c, 20, 22, 25) eine Führungseinrichtung (25) aufweist, die relativ zu der zweiten Wand (4, 6) fixiert ist und gleitend mit der Führungseinrichtung (20), die in Bezug auf die erste Wand (3, 7) fixiert ist, und/oder mit der Führungseinrichtung (2b, 2c, 22), die in Bezug auf den Hauptkörper (2) fixiert ist, im Eingriff ist.

8. Behälter nach einem der vorhergehenden Ansprüche, mit mindestens einer im Wesentlichen teleskopartigen Kopplung (20, 22, 25) zwischen dem Hauptkörper (2) und der mindestens einen Wand (3, 7; 4, 6).

9. Behälter nach Anspruch 8, wobei die Aktuatoreinrichtung (1-13, 26) einen Aktuator (10) aufweist, der in der im Wesentlichen teleskopartigen Kopplung (20, 22, 25) untergebracht oder mit dieser verbunden ist, vorzugsweise in einer Position, die im Wesentlichen koaxial dazu ist.

10. Behälter nach einem der vorhergehenden Ansprüche, wobei der Aufnahmeraum (2a) einen Boden mit Durchgangsöffnungen für ein sterilisierendes Fluid hat, wobei der Boden vorzugsweise selektiv über die mindestens eine Wand (3, 7; 4, 6) schließbar ist.

11. Behälter nach einem der vorhergehenden Ansprüche, wobei die mindestens eine Wand (3, 7; 4, 6) eine Struktur (4a, 6) hat, mit der lösbar eine Schließeinrichtung (5) verbunden ist, vorzugsweise eine manuelle Schließeinrichtung (5), etwa ein Stecker oder dergleichen.

12. Behälter nach einem der vorhergehenden Ansprüche, wobei der Aufnahmeraum in mehrere Abteile (2a) unterteilt ist, wobei insbesondere die mehreren Abteile (2a) ein oder mehrere funktionsbereite Abteile, d. h. für die Aufnahme von zu sterilisierenden Produkten (F) gestaltete Abteile, und ein oder mehrere nicht funktionsbereite Abteile, d. h. nicht für die Aufnahme von zu sterilisierenden Produkten (F) ausgebildete Abteile, aufweist, wobei ein oder mehrere nicht funktionsbereite Abteile an einer Position benachbart zu mindestens einem funktionsbereiten Abteil liegen, wobei insbesondere der Hauptkörper (2) und/oder die mindestens eine Wand (3, 7; 4, 6) so ausgebildet sind, dass das eine oder die mehreren nicht funktionsbereiten Abteile in Fluidverbindung mit der äußeren Umgebung bleiben, auch wenn der Hauptkörper (2) und die mindestens eine Wand (3, 7; 4, 6) in einer entsprechenden angenäherten Position sind.

13. Behälter nach Anspruch 11 und 12, wobei die Struktur (4a, 6) der mindestens einen Wand (4, 6) mehrere Durchgangsöffnungen (4b, 6a) aufweist, die zu entsprechenden Abteilen (2a) der mehreren Abteilungen axial ausgerichtet sind, wobei insbesondere entsprechende manuell entfernbare Stecker (5) mit den Durchgangsöffnungen (4a, 6a) verbunden sind.

14. Behälter nach einem der vorhergehenden Ansprüche mit einem Positionierelement (13), das mit der ersten Wand (3, 7) an einer im Wesentlichen stationären Position gekoppelt ist, wobei das Positionierelement (13) und die erste Wand (3, 7) eine Einrichtung für gegenseitiges Koppeln (13c, 13d, 21b, 21 c) aufweisen, wobei der Hauptkörper (2) und/oder die zweite Wand (4, 6) funktionsbereit zwischen dem Positionierelement (13) und der ersten Wand (3, 7) angeordnet sind, und wobei vorzugsweise zwischen dem Positionierelement (13) und dem Hauptkörper (2) und/oder der zweiten Wand (4, 6) eine elastische Einrichtung (11) vorgesehen ist, um den Hauptkörper (2) in eine entsprechende Position, die der ersten Wand (3, 7) angenähert ist, vorzuspannen.

15. Behälter nach einem der vorhergehenden Ansprüche, der auch ein Signalisierungselement (14), vorzugsweise zur Signalisierung, insbesondere in automatisierter Weise, einer Betätigung, die in der Betätigungsanordnung aufgetreten ist, und/oder einer relativen Verschiebung, die zwischen dem Hauptkörper (2) und der mindestens einen Wand (3, 7, 4, 6) aufgetreten ist, und/oder einer Sterilisierung, die aufgetreten ist, und/oder eines Zustand des Behälters (1), und/oder einer Eigenschaft von Produkten (F), die in dem Aufnahmeraum enthalten sind, ausgebildet ist, wobei das Signalisierungselement (14) vorzugsweise von einer ersten Position zu einer zweiten Position nach dem Übergang des Hauptkörpers (2) und der mindestens einen Wand (3, 7, 4, 6) von der angenäherten Position zu der beabstandeten Position verschiebbar ist, wobei das Signalisierungselement (14) vorzugsweise manuell betätigbar ist, um in die zuvor genannte erste Position zurückgebracht zu werden.

## Revendications

1. Contenant de stérilisation pour articles médicaux, en particulier pour articles dentaires, comprenant :
un corps principal (2) définissant un espace de confinement (2a) pour articles à stériliser (F),
au moins une paroi (3, 7 ; 4, 6) pour ouvrir et fermer l'espace de confinement (2a) au niveau d'au moins une extrémité de l'espace de confinement (2a),
des moyens de guidage (2b, 2c, 20, 22, 25) configurés pour obliger le corps principal (2) et la au moins une paroi (3, 7 ; 4, 6) à réaliser des déplacements relatifs entre une position approchée et une position espacée,
un agencement d'actionnement (10-13, 26) qui comprend des moyens d'actionneur (10) sensibles à une température de l'environnement de stérilisation,
**caractérisé en ce que** l'agencement d'actionnement (10-13, 26) est configuré pour provoquer des déplacements relatifs du corps principal (2) et de la au moins une paroi (3, 7 ; 4, 6) entre la position approchée et la position espacée.

2. Contenant selon la revendication 1, comprenant ladite première paroi (3, 7) et ladite seconde paroi (4, 6), et dans lequel :
les moyens de guidage (2b, 2c, 20, 22, 25) comprennent des moyens de guidage (20, 22) configurés pour obliger le corps principal (2) et la première paroi (3, 7) à réaliser des déplacements relatifs entre les positions approchée et espacée respectives, et
des moyens de guidage (2b, 2c, 20, 22, 25) configurés pour obliger la seconde paroi (4, 6) et le corps principal (2) à réaliser des déplacements relatifs entre les positions approchée et espacée respectives,
l'agencement d'actionnement (10-13, 26) est configuré pour provoquer le déplacement relatif entre le corps principal (2) et la première paroi (3, 7) dans les positions approchée et espacée respectives et le déplacement relatif entre la seconde paroi (4, 6) et le corps principal (2) dans les positions approchée et espacée respectives.

3. Contenant selon la revendication 1 ou la revendication 2, dans lequel l'agencement d'actionnement (10-13, 26) comprend des moyens de couplage ou de fin de course (13, 26a), configurés pour limiter la course maximum autorisée par rapport au mouvement relatif entre le corps principal (2) et la au moins une paroi (3, 7 ; 4, 6).

4. Contenant selon une ou plusieurs des revendications précédentes, dans lequel l'agencement d'actionnement (10-13, 26) comprend des moyens élastiques (11, 12) conçus pour pousser le corps principal (2) et la au moins une paroi (3, 7 ; 4, 6) dans la position approchée ou la position espacée correspondante, tels qu'au moins l'un parmi :
des moyens élastiques (11) conçus pour pousser le corps principal (2) et ladite au moins une paroi (3, 7) dans une position approchée correspondante, et
des moyens élastiques (12) conçus pour pousser le corps principal (2) et ladite au moins une paroi (4, 6) dans une position espacée correspondante.

5. Contenant selon l'une quelconque des revendications précédentes, dans lequel les moyens d'actionneur comprennent un actionneur thermique (10) comprenant :
un corps d'actionneur réalisé avec un matériau thermiquement conducteur (10a), dans lequel une cavité (10b) est définie pour contenir un matériau expansible selon la température du corps d'actionneur (10a), de préférence une cire ou une paraffine,
un arbre ou un piston plongeur (10c), ayant une extrémité faisant saillie d'une extrémité de la cavité (10b) et une seconde extrémité, à l'intérieur de la cavité (10b), l'arbre ou piston plongeur (10c) étant mobile de manière linéaire par rapport au corps d'actionneur (10a) selon l'augmentation et la réduction de volume du matériau expansible,
des moyens de fermeture (10d) pour fermer ladite extrémité de la cavité (10b) du corps d'actionneur (10a) et obtenir un joint d'étanchéité par rapport à l'arbre ou au piston plongeur (10c).

6. Contenant selon l'une des revendications précédentes, dans lequel les moyens de guidage (2b, 2c, 20, 22, 25) comprennent des moyens de guidage (20) fixes par rapport à la première paroi (3, 7) et mis en prise de manière coulissante, avec les moyens de guidage (22) fixes par rapport au corps principal (2).

7. Contenant selon l'une des revendications précédentes, dans lequel les moyens de guidage (2b, 2c, 20, 22, 25) comprennent des moyens de guidage (25) fixes par rapport à la seconde paroi (4, 6) et mis en prise, d'une manière coulissante, avec au moins l'un des moyens de guidage (20) fixes par rapport à la première paroi (3, 7) et des moyens de guidage (2b, 2c, 22) fixes par rapport au corps principal (2).

8. Contenant selon l'une des revendications précédentes, comprenant au moins un couplage sensiblement télescopique (20, 22, 25) entre le corps principal (2) et la au moins une paroi (3, 7 ; 4, 6).

9. Contenant selon la revendication 8, dans lequel les moyens d'actionneur (1-13, 26) comprennent un actionneur (10) logé dans ou associé au couplage sensiblement télescopique (20, 22, 25), de préférence dans une position sensiblement coaxiale par rapport à ce dernier.

10. Contenant selon l'une quelconque des revendications précédentes, dans lequel l'espace de confinement (2a) a un fond avec des ouvertures de passage pour un fluide de stérilisation, le fond étant de préférence sélectivement refermable via la au moins une paroi (3, 7 ; 4, 6).

11. Contenant selon l'une quelconque des revendications précédentes, dans lequel la au moins une paroi (3, 7 ; 4, 6) a une structure (4a, 6) à laquelle on associe, de manière amovible, des moyens de fermeture (5), de préférence des moyens de fermeture manuels, tel que des bouchons ou similaires.

12. Contenant selon l'une quelconque des revendications précédentes, dans lequel l'espace de confinement est divisé en une pluralité de compartiments (2a), où de préférence la pluralité de compartiments (2a) comprend un ou plusieurs compartiments opérationnels, c'est-à-dire conçus pour loger des articles (F) à stériliser, et un ou plusieurs compartiments non opérationnels, c'est-à-dire non conçus pour loger des articles (F) à stériliser, les un ou plusieurs compartiments non opérationnels étant dans une position adjacente à au moins un compartiment opérationnel, encore de préférence le corps (2) et/ou la au moins une paroi (3, 7 ; 4, 6) étant configurés de sorte que les un ou plusieurs compartiments non opérationnels restent en communication de fluide avec l'environnement externe, également lorsque le corps principal (2) et la au moins une paroi (3, 7 ; 4, 6) sont dans une position approchée correspondante.

13. Contenant selon les revendications 11 et 12, dans lequel la structure (4a, 6) de la au moins une paroi (4, 6) a une pluralité d'ouvertures débouchantes (4b, 6a) axialement alignées par rapport aux compartiments (2a) respectifs de ladite pluralité, on associe de préférence aux ouvertures débouchantes (4a, 6a), des bouchons (5) manuellement amovibles, respectifs.

14. Contenant selon l'une des revendications précédentes, comprenant un élément de positionnement (13) couplé à la première paroi (3, 7) dans une position sensiblement fixe, l'élément de positionnement (13) et la première paroi (3, 7) ayant des moyens pour le couplage réciproque (13c, 13d, 21b, 21c), au moins l'un parmi le corps principal (2) et la seconde paroi (4, 6) étant placée de manière opérationnelle entre l'élément de positionnement (13) et la première paroi (3, 7), entre l'élément de positionnement (13) et au moins l'un parmi le corps principal (2) et la seconde paroi (4, 6), on prévoit de préférence des moyens élastiques (11) pour pousser le corps principal (2) dans une position approchée respective par rapport à la première paroi (3, 7).

15. Contenant selon l'une des revendications précédentes, comprenant également un élément de signalement (14), de préférence conçu pour signaler, en particulier d'une manière automatique, au moins l'un parmi un actionnement qui s'est produit de l'agencement d'actionnement, un déplacement relatif qui s'est produit entre le corps principal (2) et la au moins une paroi (3, 7, 4, 6), une stérilisation qui s'est produite, une condition du contenant (1), une caractéristique des articles (F) contenus dans l'espace de confinement, l'élément de signalement (14) pouvant être de préférence déplacé d'une première position à une seconde position suite au passage du corps principal (2) et de la au moins une paroi (3, 7, 4, 6) de la position approchée à la position espacée, l'élément de signalement (14) pouvant, encore de préférence, être actionné manuellement pour être ramené dans la première position mentionnée ci-dessus.
